# EUROPÄISCHE PATENTSCHRIFT

(11) **EP 1 530 971 B1**
(45) Veröffentlichungstag und Bekanntmachung des Hinweises auf die Patenterteilung: **06.02.2008**
(21) Anmeldenummer: 04024730.6
(22) Anmeldetag: 16.10.2004
(51) Int. Cl.: A61K 36/00, A61P 1/02

(54) **Zusammensetzung zur Behandlung von Zahn- und Zahnfleischerkrankungen**
Composition for the treatment of tooth and gum diseases
Composition pour le traitment des maladies de la dent et de la gencive

(30) Priorität: 17.11.2003 DE 10353743; 06.12.2003 DE 10357110
(43) Veröffentlichungstag der Anmeldung: 18.05.2005
(73) Patentinhaber: Maria Clementine Martin Klosterfrau Vertriebsgesellschaft mbH, 50670 Köln (DE)
(72) Erfinder: Greve, Harald, Dr., 40545 Düsseldorf (DE); Greve, Rainer, Dr., 23795 Bad Segeberg (DE)
(74) Vertreter: Gesthuysen, von Rohr & Eggert

(56) Entgegenhaltungen:
- PENABAD, T. ET AL.: "Toxicologia Subcronica Bucal Del Extracto Fluido De Plantago Lanceolata" REV CUBANA PLANT MED, Bd. 1, Nr. 2, Mai 1996 (1996-05), Seiten 24-26, XP002315271
- MONOGRAPHIE KOMMISSION E: DATEI-NR. 00741: "Monographie: Plantaginis lanceolatae herba" BUNDESANZEIGER, Nr. 223, 30. November 1985 (1985-11-30), XP002315272 Gefunden im Internet: URL:www.heilpflanzen-welt.de>

## Beschreibung

Die vorliegende Erfindung betrifft die Verwendung von Eisenkraut und Spitzwegerich bzw. deren Pflanzenteilen und/oder Extrakten zur Herstellung von geeigneten Zubereitungen, die zur positiven Beeinflussung der Mundflora des Menschen bestimmt sind, insbesondere zur prophylaktischen und/oder kurativen topischen Behandlung von Zahn- und Zahnfleischerkrankungen, wie Zahnkaries, Parodontitis, Gingivitis, Plaque (Zahnbelag) und die damit verbundenen Folgen, insbesondere Mundgeruch.

Insbesondere betrifft die vorliegende Erfindung die Verwendung von Eisenkraut und Spitzwegerich bzw. deren Extrakten bzw. Inhaltsstoffen sowie eine diese enthaltende pharmazeutische Zusammensetzung zur prophylaktischen und kurativen topischen Behandlung von Zahn- und Zahnfleischerkrankungen, wie Zahnkaries, Parodontitis, Gingivitis, Plaque (Zahnbelag) und damit im Zusammenhang stehenden Symptomen bzw. Erscheinungen, wie Mundgeruch.

Eine negative Verschiebung der Mundflora des Menschen kann sich sehr unangenehm äußern, insbesondere als übler Mundgeruch bis hin zu Zahn- und Zahnfleischerkrankungen, wie Plaque (Zahnbelag), Zahnkaries, Parodontitis und Gingivitis, wobei in diesem Zusammenhang insbesondere die Vermehrung folgender Keime eine besondere Rolle spielt: Streptococcus mutans, Streptococcus sobrinus, Streptococcus sanguinus, Streptococcus salivarius, Porphyromonas gingivalis, Fusobacterium nucleatum und Actinomyces viscosus.

Bislang werden zur Bekämpfung von Mundgeruch, Plaque, Karies, Gingivitis und Parodontitis chemisch-synthetische Antiseptika, wie z. B. Hexetidin, Dequaliniumchlorid, Chlorhexidin und etherische Öle, ohne besonders großen Erfolg eingesetzt.

Penabad , T. et al .: Toxicologica Subcromica Bucal Del Extracto Fluido De Plantago Lanceolata; Rev. Cubana Plant Med ; Band1 , Niv. 2 , Mai 1996, Seiten 24-26 beschreibt die Verwendung von Spitzwegerich zur Behandlung von Mundhöhlenerkrankungen wie Gingivitis.

Ebenso wird die Verwendung von Spitzwegerich zur Behandlung entzündlicher Veränderungen der Mund - und Raschenschleimhaut in der Monographie Kommision E : Datei-Nr. 00741, Bandesanzeiger , Nr. 223, 30. November 1985, beschrieben.

Alternativ werden auch bestimmte anorganische Verbindungen, wie beispielsweise Aluminiumchlorid, Aminfluoride oder Zinn-(II)-fluorid, mit mehr oder weniger großem Erfolg eingesetzt. Bei diesen Substanzen kann die längerfristige Anwendung zur Verfärbung des Zahnschmelzes führen, und z. B. kann ein Verschlucken dieser Substanzen beim Gurgeln zu unerwünschten Begleiterscheinungen führen.

Breit anwendbare, stark wirksame und unbedenkliche Mittel auf pflanzlicher Basis stehen bis heute zur effektiven Bekämpfung von Zahn- und Zahnfleischerkrankungen, wie Zahnkaries, Parodontitis, Gingivitis, Plaque (Zahnbelag) und damit im Zusammenhang stehenden Symptomen bzw. Erscheinungen, wie Mundgeruch, nicht zur Verfügung.

Aufgabe der vorliegenden Erfindung ist somit die Bereitstellung eines breit anwendbaren, gut verträglichen Mittels zur Prophylaxe bzw. wirksamen Therapie von Zahn- und Zahnfleischerkrankungen, insbesondere Zahnkaries, Parodontitis, Gingivitis, Plaque (Zahnbelag) und damit im Zusammenhang stehenden Symptomen bzw. Erscheinungen, wie Mundgeruch, welches die zuvor geschilderten Probleme des Standes der Technik zumindest weitgehend vermeidet.

Die Anmelderin hat nun herausgefunden, daß die vorgenannte Aufgabe in vollkommen überraschender Weise durch die Verwendung von Eisenkraut und Spitzwegerich in geeigneten Zubereitungen gelöst werden kann.

Gegenstand der vorliegenden Erfindung gemäß einem ersten Aspekt der Erfindung ist somit eine pharmazeutische Zusammensetzung zur prophylaktischen und/oder kurativen topischen Behandlung von Zahn- und Zahnfleischerkrankungen (z. B. Zahnkaries, Gingivitis, Parodontitis und Plaque bzw. Zahnbelag sowie damit im Zusammenhang stehende Symptome bzw. Erscheinungen, wie Mundgeruch), welche - neben mindestens einem pharmazeutischen Träger - Eisenkraut und Spitzwegerich oder deren Inhaltsstof fe oder Extrakte in jeweils pharmazeutisch wirksamen Mengen enthält.

Gegenstand der vorliegenden Erfindung gemäß einem zweiten Aspekt der Erfindung ist somit die Verwendung von Eisenkraut und Spitzwegerich bzw. deren jeweiliger Inhaltsstoffe oder Extrakte zur prophylaktischen und/oder kurativen topischen Behandlung von Zahn- und Zahnfleischerkrankungen, insbesondere Zahnkaries, Gingivitis, Parodontitis und Plaque (Zahnbelag) sowie damit im Zusammenhang stehenden Symptomen bzw. Erscheinungen, wie Mundgeruch, bzw. zur Herstellung eines pharmazeutischen Mittels zur prophylaktischen und/oder kurativen topischen Behandlung von Zahn- und Zahnfleischerkrankungen, insbesondere Zahnkaries, Gingivitis, Parodontitis und Plaque (Zahnbelag) sowie damit im Zusammenhang stehenden Symptomen bzw. Erscheinungen, wie Mundgeruch.

Das Eisenkraut und der Spitzwegerich sind unter anderem in Europa heimische Pflanzen, die in Form ihrer Kräuter seit geraumer Zeit für andere Indikationen angewendet werden. Zur erfindungsgemäßen Verwendung können alle Teile der beiden Pflanzen herangezogen werden, also Wurzel, Sprosse, Blätter und Früchte. Bevorzugt wird allerdings der gesamte oberirdische Teil verwendet (Herba). Die beiden Pflanzen sind handelsüblich und als solche leicht beschaffbar.

Das Eisenkraut (Herba Verbenae) mit der Stammpflanze Verbena officinalis, Verbenaceae, ist als Droge nur wenig gebräuchlich und wird traditionell beispielsweise als Adstringens und als Bittermittel verwendet. Der Droge werden darüber hinaus diuretische, galactagoge und uteruskontrahierende Wirkungen zugeschrieben. Ethanolische Extrakte zeigen antibakterielle und antivirale Effekte. Die pharmazeutisch angewandten Pflanzenteile des Eisenkrauts sind insbesondere die zur Blütezeit gesammelten Blätter und die oberen Stengelabschnitte. Inhaltsstoffe des Eisenkrauts sind insbesondere Verbenalin und andere Iridoidglykoside, Verbascosid und andere Phenylethanoidglykoside, Luteolin-7-0-diglucuronid und andere Flavonoide, darunter auch methoxylierte Flavone, ferner Stachyose, β-Sitosterol und Triterpene. Für das Verbenalin wie auch für andere Iridoide konnten schwach parasympathomimetische, antiphlogistische und analgetische Effekte nachgewiesen werden.

Der Spitzwegerich bzw. dessen Kraut, Herba Plantaginis lanceolatae (angustifoliae), Plantaginaceae, wird in der Volksheilkunde zur Sekretolyse bei Katarrhen der oberen Luftwege eingesetzt. Bekannt ist insbesondere die antitussive Wirkung mit bronchospasmolytischen und expektorierenden Effekten. Der Preßsaft des frischen Krauts soll sich aufgrund seiner antibakteriellen wie antiinflammatorischen Wirkung auch äußerlich als Wundheilmittel und gegen Insektenstiche eignen. Die pharmazeutisch angewandten Pflanzenteile sind insbesondere das Spitzwegerichkraut, Herba Plantaginis lanceolatae, die zur Blütezeit geernteten und schnell getrockneten oberirdischen Teile. Inhaltsstoffe des Spitzwegerichs sind insbesondere Aucubin, Catalpol und andere Iridoidglykoside, Acteosid und weitere Phenylethanoide, Phenolcarbonsäuren, wie z. B. Kaffeesäure, Ferulasäure, ferner Flavonoide, Gerbstoffe, Schleimpolysaccharide, Kieselsäure, etherische Öle und Vitamin C.

Vorteilhafterweise enthält die erfindungsgemäße pharmazeutische Zusammensetzung Eisenkraut und Spitzwegerich bzw. deren Inhaltsstoffe oder Extrakte als alleinige Wirkstoff z. B. in Form zerkleinerter, insbesondere pulverisierter Pflanzenbestandteile und/oder in Form deren extrahierter Inhaltsstoffe oder in Form von Extrakten.

Dennoch ist es nicht ausgeschlossen, daß die erfindungsgemäße pharmazeutische Zusammensetzung daneben noch weitere Wirkstoffe und/oder Inhaltsstoffe enthält. Beispielsweise kann die erfindungsgemäße pharmazeutische Zusammensetzung bzw. Zubereitung auch andere Wirkstoffe oder Inhaltsstoffe bzw. Gemische weiterer Wirkstoffe oder Inhaltsstoffe enthalten, die sowohl pflanzlichen als auch nichtpflanzlichen Ursprungs sein können und die Wirkung des Eisenkrauts und des Spitzwegerichs nicht beeinträchtigen oder sogar deren Wirkung noch verstärken. Darüber hinaus können Stoffe oder Stoffgemische zugesetzt sein, die den Geschmack und/oder den Geruch sowie die technologische Anwendung der erfindungsgemäßen pharmazeutischen Zusammensetzung verbessern.

Beispielsweise kann die erfindungsgemäße pharmazeutische Zusammensetzung Eisenkraut und Spitzwegerich in Form ihrer jeweiligen Extrakte, insbesondere in Form von Flüssig- oder Trockenextrakten, enthalten, wobei das bevorzugte Droge/Extrakt-Verhältnis 50 : 1 bis 1 : 1, besonders bevorzugt 10 : 1 bis 1 : 0,2, beträgt. Beispielsweise kann die pharmazeutische Zusammensetzung nach der vorliegenden Erfindung Eisenkraut und Spitzwegerich in Form flüssiger Extrakte (z. B. in Form alkoholischer oder alkoholischwäßriger Extrakte, vorzugsweise in Form ethanolischer oder ethanolisch-wäßriger Extrakte) oder aber in Form fester Extrakte enthalten.

Dabei kann die pharmazeutische Zusammensetzung nach der vorliegenden Erfindung flüssig, gelartig, pastös, salbenförmig oder fest sein, je nach beabsichtigter Applikation. Beispielsweise kann die erfindungsgemäße pharmazeutische Zusammensetzung z. B. in Form von (Lutsch-/Kau-)Tabletten oder (Lutsch-/Kau-)Pastillen oder aber in Form einer zur topischen Applikation im Mund-/Rachenraum verwendbaren Lösung oder Dispersion, insbesondere Spül- und/oder Gurgellösung, oder aber in Form von zur topischen Applikation im Mund-/Rachenraum verwendbaren Salben, Cremes, Gele oder Pasten, insbesondere Zahnpasten oder Zahncremes, vorliegen.

Die Mengen an Eisenkraut und Spitzwegerich oder deren Inhaltsstoffen oder Extrakten in der erfindungsgemäßen pharmazeutischen Zusammensetzung können in weiten Bereichen variieren. Im allgemeinen kann die erfindungsgemäße pharmazeutische Zusammensetzung, jeweils berechnet auf die Masse des pflanzlichen Ausgangsmaterials und bezogen auf die pharmazeutische Zusammensetzung, Eisenkraut und Spitzwegerich bzw. deren Inhaltsstoffe oder Extrakte, in Mengen von mindestens 0,1 Gew.-%, insbesondere mindestens 1 Gew.-%, vorzugsweise mindestens 5 Gew.-%, besonders bevorzugt mindestens 10 Gew.-%, und in Mengen von bis zu 50 Gew.-%, insbesondere von bis zu 75 Gew.-%, vorzugsweise von bis zu 90 Gew.-%, besonders bevorzugt von bis zu 95 Gew.-% oder sogar mehr, enthalten. Dennoch kann es gegebenenfalls erforderlich sein, von den genannten Mengen abzuweichen, sofern die therapeutischen Gegebenheiten dies erfordern.

Neben den eigentlichen Wirkstoffen auf Basis von Eisenkraut undSpitzwegerich kann die pharmazeutische Zusammensetzung nach der vorliegenden Erfindung außerdem übliche pharmazeutische Zusatz- und/oder Hilfsstoffe, insbesondere Füll-, Streck-, Binde-, Netz-, Stabilisierungs-, Färbe-, Puffer-, Riech-, Geschmacks- und/oder Konservierungsstoffe, enthalten.

Die beiden Pflanzen bzw. Pflanzenteile - auch in zerkleinerter Form - bis hin zum Pulver könzur erfindungsgemäßen Verwendung als solche eingesetzt werden. Bevorzugt ist allerdings die Verwendung in Form von Extrakten bzw. Inhaltsstoffen der beiden Pflanzen.

Wie zuvor beschrieben, können die Extrakte fest bzw. trocken oder aber flüssig sein. Flüssige Extrakte können neben den pflanzlichen Wirkstoffen flüssige Auszugsmittel, die dem Fachmann an sich bekannt sind, enthalten. Bevorzugt sind Wasser, Alkohole, insbesondere Trinkalkohol (Ethanol) oder Ethanol/Wasser-Gemische. Feste bzw. trockene Extrakte können neben den pflanzlichen Wirk- bzw. Inhaltsstoffen insbesondere inerte Feststoffe, wie beispielsweise Siliciumdioxid oder Xylit, enthalten.

Die Extrakte weisen ein bevorzugtes Droge/Extrakt-Verhältnis von 50 : 1 bis 1 : 1, besonders bevorzugt von 10 : 1 bis 1 : 0,2 auf. Die Herstellung der Extrakte kann in einer für den Fachmann an sich bekannten Weise erfolgen, z. B. derart, daß mit Hilfe geeigneter flüssiger Auszugsmittel, bevorzugt Ethanol/Wasser, beispielsweise aus 1 Teil getrockneter oder frischer Pflanzenmasse (Krautmasse) 0,2 bis 100 Teile, vorzugsweise 1 Teil flüssiger oder 0,1 Teile trockener Extrakt gewonnen werden. Dabei kann die zur Erzielung des jeweils gewünschten Droge/Extrakt-Verhältnisses erforderliche Extraktmasse bei flüssigen Extrakten durch Direktextraktion mit oder ohne nachträgliches Einengen, bei Trockenextrakten durch Eindampfen flüssiger Auszüge zur Trocknung und anschließendes Vermischen mit inerten Feststoffen eingestellt werden. Dies ist dem Fachmann an sich bekannt, so daß es diesbezüglich keiner weitergehenden Ausführungen bedarf.

Zubereitungen, die Eisenkraut und Spitzwegerich oder deren Extrakte enthalten, können flüssig, gelartig oder pastös sein und neben den pflanzlichen Bestandteilen bzw. Extraktivstoffen die in der Herstellungstechnologie üblichen, unbedenklichen und mit dem Eisenkraut und Spitzwegerichkraut verträglichen Verdünnungsmittel und andere Hilfsstoffe enthalten.

Als besonders geeignet zur Prophylaxe und Bekämpfung der Plaquebildung, Gingivitis, Karies und Parodontitis hat sich ein Gurgelspülkonzept erwiesen, das etwa 2,5 % eines Extraktes enthält, der etwa 60 % Ethanol (Vol-%) enthält. Von dieser Zubereitung reichen etwa 20 Tropfen auf ein halbes Glas Wasser (ca. 50 ml), um eine ausreichende Keimhemmung zu erzielen.

Um einen ausreichenden Effekt bei den halbfesten Formen, wie z. B. Zahnpasta oder Mundgelen, zu erzielen, können diese ebenfalls etwa 2,5 % eines Extraktes enthalten, der etwa 80 % Ethanol (Vol-%) enthält.

Bei Lutsch- und Kautabletten bzw. -pastillen ist es bevorzugt, daß diese etwa 250 mg Trockenextrakt je Einheit enthalten. Im günstigsten Fall ist dieser Trockenextrakt aus dem Flüssigkeitsextrakt durch Einengung hervorgegangen, der ursprünglich ein Droge/Extrakt-Verhältnis von etwa 1 : 1 aufweist und einen alkoholischen Anteil von etwa 80 % (Vol-%).

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand der folgenden Ausführungsbeispiele veranschaulicht, welche die vorliegende Erfindung jedoch keinesfalls beschränken.

### Ausführungsbeispiele:

### Beispiel 1

Zur Herstellung eines Flüssigextraktes aus Eisenkraut mit einem Droge/Extrakt-Verhältnis von 1 : 1 werden 100 g des im Handel erhältlichen, getrockneten und geschnittenen Eisenkrauts nach dem im DAB 9, Seite 800 in der Monographie "Extrakte" beschriebenen Verfahren durch Perkolation extrahiert. Die Droge wird dabei zuerst durch Vermahlen grob gepulvert, mit 30 g Ethanol (70 Vol.-%) in einer offenen Schale gleichmäßig durch Feuchte bedeckt, wo sie zwei Stunden zum Vorquellen stehengelassen wird. Danach wird gesiebt, das Gemisch in den vom DAB 9 vorgeschriebenen Perkolator gefüllt und nach dem dort angegebenen Verfahren die Perkolation mit 70%igem Ethanol (Vol.-%) durchgeführt. Nachdem 85 g Perkolat abgetropft sind, ist die Perkolation beendet. Dann wird der Ablaufhahn geschlossen, die weitere Aufgabe von Ethanol (70 Vol.-%) unterbrochen und der Drogenrückstand abgepreßt. Die Preßflüssigkeit wird filtriert, am Rotationsverdampfer unter Vakuum eingeengt und zu dem Perkolat gegeben. Nach mehrtätigem Stehenlassen im geschlossenen Gefäß bei etwa 5 bis 10 °C wird das Produkt auf Klarheit geprüft und gegebenenfalls filtriert. Man erhält einen Flüssigextrakt auf Basis von Eisenkraut mit einem Droge/Extrakt-Verhältnis von 1 : 1.

Der Extrakt eignet sich zur Herstellung eines Mundspül-/Gurgelkonzentrates, das etwa 2,5 % des Extraktes enthält, der etwa 60 % Ethanol (Vol.-%) und den Rest Wasser enthält.

### Beispiel 2:

Beispiel 1 wird wiederholt, jedoch mit der Ausnahme, daß das Eisenkraut vollständig durch Spitzwegerichkraut ersetzt wird.

### Beispiel 3:

Beispiel 1 wird wiederholt, jedoch mit der Ausnahme, daß 50 g des Eisenkrauts durch Spitzwegerichkraut ersetzt wird. Es resultiert ein Extrakt auf Basis von Eisenkraut und Spitzwegerich in Kombination. Der Extrakt bzw. das auf Basis dieses Extrakts hergestellte Mundspül-/Gurgelkonzentrat eignen sich gleichermaßen zur Behandlung von Zahn- und Zahnfleischerkrankungen.

### Beispiele 4 bis 6:

Beispiele 1 bis 3 werden dahingehend modifiziert, daß ausgehend von den in den Beispielen 1 bis 3 beschriebenen wäßrig-alkoholischen Extrakten jeweils Trockenextrakte hergestellt werden, die anschließend in an sich bekannter Weise mit den hierfür üblichen Träger- und Hilfsstoffen zu Lutsch- bzw. Kautabletten verarbeitet werden (Droge/Extrakt-Verhältnis jeweils 1 : 1, jeweils 250 mg Trockenextrakt je Einheit bzw. Tablette).
Die auf diese Weise hergestellten Lutsch- bzw. Kautabletten eignen sich gleichermaßen zur Behandlung von Zahn- und Zahnfleischerkrankungen der vorgenannten Art.

### Beispiel 7:

Verschiedene flüssige ethanolische Extrakte auf Basis von Eisenkraut ("V30", "V50" und "V80") und auf Basis von Spitzwegerich ("P30", "P50" und "P80") sowie auf Basis von Eisenkraut plus Spitzwegerich ("V80 plus P80") mit jeweils unterschiedlichen Konzentrationen wurden im Hinblick auf ihre Wirksamkeit in bezug auf die Hemmung des Wachstums bestimmter pathogener Keime der Mundflora getestet.

| | | |
|---|---|---|
| Proben: | Fluidextrakt V30 | Ethanol-Istgehalt: 24,3 % |
| | Fluidextrakt V50 | Ethanol-Istgehalt: 41,3 % |
| | Fluidextrakt V80 | Ethanol-Istgehalt: 63,3 % |
| | Fluidextrakt P30 | Ethanol-Istgehalt: 20,2 % |
| | Fluidextrakt P50 | Ethanol-Istgehalt: 38,6 % |
| | Fluidextrakt P80 | Ethanol-Istgehalt: 63,8 % |
| | 1 : 1-Gemisch V80 plus P80 (Eisenkraut plus Spitzwegerich) | |

In diesen Untersuchungen wurden die sogenannten minimalen Hemmkonzentrationen (MHK) von sechs einzelnen Fluidextrakten sowie einem Fluidextraktgemisch (1 : 1) von V80 und P80 bestimmt, d. h. es wurden die niedrigsten Konzentrationen dieser Extrakte bzw. des Gemisches ermittelt, die das Wachstum bestimmter Bakterien hemmen. Es wurden dazu Bakterien der Mundflora des Menschen ausgewählt, die Infektionen der Zähne und des Zahnfleischs auslösen können (siehe Tabelle 1). Dazu gehört beispielsweise das Bakterium *Streptococcus mutans*, das hauptverantwortlich für die Karies-Entstehung ist.

Die Testdurchführung erfolgte in Anlehnung an die DIN-Methoden 58940 und 58944. Es wurden jeweils zehn Konzentrationen ausgehend von der höchsten Konzentration (10 %) der verschiedenen Fluidextrakte getestet.

In den Tabellen 2 bis 8 sind die Ergebnisse dargestellt. Als "Minimale Hemmkonzentration" (MHK) wird die niedrigste Wirkstoffkonzentration angesehen, bei der makroskopisch kein Wachstum der getesteten Bakterien zu beobachten ist. Die Ermittlung der MHK ist allgemein mit einer Fehlergrenze von bis zu zwei Verdünnungsstufen behaftet.

**Tabelle 1: Ausgewählte Keime der Mundflora und ihr pathogenes Potential**

| Keim | pathogenes Potential |
|---|---|
| *Streptococcus mutans* | Karies-Erreger |
| *Streptococcus sobrinus* | Karies-Erreger |
| *Streptococcus sanguis* | indirekt, Zahnbelag (Plaque) |
| *Streptococcus salivarius* | selten pathogen (Karies) |
| *Porphyromonas gingivalis* | Parodontose |
| *Fusobacteritim nucleatum* | dentogene Eiterungen, Parodontose |
| *Actinomyces viscosus* | Karies, Parodontose |

Für alle Fluidextrakte war unter den beschriebenen Versuchsbedingungen eine das Wachstum von Bakterien hemmende Wirkung zu beobachten. Im Vergleich der Fluidextrakte V30, V50 und V80 bzw. P30, P50 und P80 waren die niedrigsten MHK für fast alle Bakterien mit dem Fluidextrakt V80 bzw. P80 zu beobachten. D. h. die Fluidextrakte V80 und P80 stellten die wirksamsten Extrakte bezüglich einer Hemmung des Wachstums der getesteten Bakterien unter den angewendeten Versuchsbedingungen dar.

Die niedrigsten wirksamen Konzentrationen für die getesteten Bakterien lagen in folgenden Bereichen:
V80: 0,63 - 2,5 %; für den Karies-Erreger Streptococcus mutans bei 2,5 %
P80: 0,63 - 5 %; für den Karies-Erreger Streptococcus mutans bei 5 %

Als noch wirksamer als die Einzelextrakte stellte sich ein eine Zubereitung gemäß Anspruch 1 im 1 : 1-Gemisch der Fluidextrakte V80 plus P80 (Fluidextrakt auf Basis von Eisenkraut und Spitzwegerich) heraus (siehe Tabelle 8). Hier lagen die kleinsten wirksamen Konzentrationen für die getesteten Bakterien in einem deutlich niedrigeren Bereich (0,16 - 0,63 % des 1 : 1-Gemisches). Für *Streptococcus mutans* lag die minimale Hemmkonzentration bei 0,31 %, also eine ca. 10fach niedrige Konzentration als die einzelnen Fluidextrakte führte noch zu einer Hemmung des Wachstums dieses Bakteriums unter den gewählten Versuchsbedingungen.

Die höhere Wirksamkeit des 1 : 1-Gemisches von V80 plus P80 weist auf einen synergistischen Effekt hin, d. h. die Fluidextrakte beider Kräuter in Kombination (Eisenkraut und Spitzwegerich) verstärken sich gegenseitig in ihrer Wirksamkeit.

**Tabelle 2: Versuchsergebnisse der Ermittlung der MHK der Probe "Fluidextrakt V30"**

| Endkonzentration in % | 10 | 5 | 2,5 | 1,25 | 0,63 | 0,31 | 0,16 | 0,08 | 0,04 | 0,02 | MHK in % |
|---|---|---|---|---|---|---|---|---|---|---|---|
| Testkeim | | | | | | | | | | | |
| *Actinomyces viscosus* | - - | - - | + + | + + | + + | + + | + + | + + | + + | + + | 5 |
| *Fusobacterium nucleatum* | - - | + + | + + | + + | + + | + + | + + | + + | + + | + + | 10 |
| *Porphyromonas gingivalis* | - - | + + | + + | + + | + + | + + | + + | + + | + + | + + | 10 |
| *Streptococcus mutans* | - - | + + | + + | + + | + + | + + | + + | + + | + + | + + | 10 |
| *Streptococcus salivarius* | - - | + + | + + | + + | + + | + + | + + | + + | + + | + + | 10 |
| *Streptococcus sanguinis* | - - | + + | + + | + + | + + | + + | + + | + + | + + | + + | 10 |
| *Streptococcus sobrinus* | - - | + + | + + | + + | + + | + + | + + | + + | + + | + + | 10 |

| | | | | | | | | | | | |
|---|---|---|---|---|---|---|---|---|---|---|---|
| + = Wachstum, - = kein Wachstum | | | | | | | | | | | |

**Tabelle 3: Versuchsergebnisse der Ermittlung der MHK der Probe "Fluidextrakt V50"**

| Endkonzentration in % | 10 | 5 | 2,5 | 1,25 | 0,63 | 0,31 | 0,16 | 0,08 | 0,04 | 0,02 | MHK in % |
|---|---|---|---|---|---|---|---|---|---|---|---|
| Testkeim | | | | | | | | | | | |
| *Actinomyces viscosus* | - - | - - | - - | - - | - - | + + | + + | + + | + + | + + | 0,63 |
| *Fusobacterium nucleatum* | - - | - - | - - | - - | + + | + + | + + | + + | + + | + + | 1,25 |
| *Porphyromo- nas gingivalis* | - - | - - | - - | - - | - - | + + | + + | + + | + + | + + | 0,63 |
| *Streptococcus mutans* | - - | - - | + + | + + | + + | + + | + + | + + | + + | + + | 5 |
| *Streptococcus salivarius* | - - | - - | - - | + + | + + | + + | + + | + + | + + | + + | 2,5 |
| *Streptococcus sanguinis* | - - | - - | - - | + + | + + | + + | + + | + + | + + | + + | 2,5 |
| *Streptococcus sobrinus* | - - | - - | - - | + + | + + | + + | + + | + + | + + | + + | 2,5 |

| | | | | | | | | | | | |
|---|---|---|---|---|---|---|---|---|---|---|---|
| + = Wachstum, - = kein Wachstum | | | | | | | | | | | |

**Tabelle 4: Versuchsergebnisse der Ermittlung der MHK der Probe "Fluidextrakt V80"**

| Endkonzentration in % | 10 | 5 | 2,5 | 1,25 | 0,63 | 0,31 | 0,16 | 0,08 | 0,04 | 0,02 | MHK in % |
|---|---|---|---|---|---|---|---|---|---|---|---|
| Testkeim | | | | | | | | | | | |
| *Actinomyces viscosus* | - - | - - | - - | - - | + - | + + | + + | + + | + + | + + | 1,25 |
| *Fusobacterium nucleatum* | - - | - - | - - | - - | - - | + + | + + | + + | + + | + + | 0,63 |
| *Porphyromo- nas gingivalis* | - - | - - | - - | - - | - - | + + | + + | + + | + + | + + | 0,63 |
| *Streptococcus mutans* | - - | - - | - - | + + | + + | + + | + + | + + | + + | + + | 2,5 |
| *Streptococcus salivarius* | - - | - - | - - | - - | - - | + + | + + | + + | + + | + + | 0,63 |
| *Streptococcus sanguinis* | - - | - - | - - | - - | + - | + + | + + | + + | + + | + + | 1,25 |
| *Streptococcus sobrinus* | - - | - - | - - | - - | - - | + + | + + | + + | + + | + + | 0,63 |

| | | | | | | | | | | | |
|---|---|---|---|---|---|---|---|---|---|---|---|
| + = Wachstum, - = kein Wachstum | | | | | | | | | | | |

**Tabelle 5: Versuchsergebnisse der Ermittlung der MHK der Probe "Fluidextrakt P30"**

| Endkonzentration in % | 10 | 5 | 2,5 | 1,25 | 0,63 | 0,31 | 0,16 | 0,08 | 0,04 | 0,02 | MHK in % |
|---|---|---|---|---|---|---|---|---|---|---|---|
| Testkeim | | | | | | | | | | | |
| *Actinomyces viscosus* | - - | + - | + + | + + | + + | + + | + + | + + | + + | + + | 10 |
| *Fusobacterium nucleatum* | - + | + + | + + | + + | + + | + + | + + | + + | + + | + + | > 10 |
| *Porphyromonas gingivalis* | - - | - - | + + | + + | + + | + + | + + | + + | + + | + + | 5 |
| *Streptococcus mutans* | - - | - - | + + | + + | + + | + + | + + | + + | + + | + + | 5 |
| *Streptococcus salivarius* | - - | - - | + + | + + | + + | + + | + + | + + | + + | + + | 5 |
| *Streptococcus sanguinis* | - - | - + | + + | + + | + + | + + | + + | + + | + + | + + | 10 |
| *Streptococcus sobrinus* | - - | - - | + + | + + | + + | + + | + + | + + | + + | + + | 5 |

| | | | | | | | | | | | |
|---|---|---|---|---|---|---|---|---|---|---|---|
| + = Wachstum, - = kein Wachstum | | | | | | | | | | | |

**Tabelle 6: Versuchsergebnisse der Ermittlung der MHK der Probe "Fluidextrakt P50"**

| Endkonzentration in % | 10 | 5 | 2,5 | 1,25 | 0,63 | 0,31 | 0,16 | 0,08 | 0,04 | 0,02 | MHK in % |
|---|---|---|---|---|---|---|---|---|---|---|---|
| Testkeim | | | | | | | | | | | |
| *Actinomyces viscosus* | - - | - - | - - | - - | + + | + + | + + | + + | + + | + + | 1,25 |
| *Fusobacterium nuc*/*eatum* | - - | - - | - - | + + | + + | + + | + + | + + | + + | + + | 2,5 |
| *Porphyromonas gingivalis* | - - | - - | - - | - - | + + | + + | + + | + + | + + | + + | 1,25 |
| *Streptococcus mutans* | - - | - - | + + | + + | + + | + + | + + | + + | + + | + + | 5 |
| *Streptococcus salivarius* | - - | - - | - - | + + | + + | + + | + + | + + | + + | + + | 2,5 |
| *Streptococcus sanguinis* | - - | - - | - - | + + | + + | + + | + + | + + | + + | + + | 2,5 |
| *Streptococcus sobrinus* | - - | - - | - - | + + | + + | + + | + + | + + | + + | + + | 2,5 |

| | | | | | | | | | | | |
|---|---|---|---|---|---|---|---|---|---|---|---|
| + = Wachstum, - = kein Wachstum | | | | | | | | | | | |

**Tabelle 7: Versuchsergebnisse der Ermittlung der MHK der Probe "Fluidextrakt P80"**

| Endkonzentration in % | 10 | 5 | 2,5 | 1,25 | 0,63 | 0,31 | 0,16 | 0,08 | 0,04 | 0,02 | MHK in % |
|---|---|---|---|---|---|---|---|---|---|---|---|
| Testkeim | | | | | | | | | | | |
| *Actinomyces viscosus* | - - | - - | - - | - - | - - | + + | + + | + + | + + | + + | 0,63 |
| *Fusobacterium nucleatum* | - - | - - | - - | - - | + + | + + | + + | + + | + + | + + | 1,25 |
| *Porphyromonas gingivalis* | - - | - - | - - | - - | - - | + + | + + | + + | + + | + + | 0,63 |
| *Streptococcus mutans* | - - | - - | + + | + + | + + | + + | + + | + + | + + | + + | 5 |
| *Streptococcus salivarius* | - - | - - | - - | - - | + - | + + | + + | + + | + + | + + | 1,25 |
| *Streptococcus sanguinis* | - - | - - | - - | - - | - - | + + | + + | + + | + + | + + | 0,63 |
| *Streptococcus sobrinus* | - - | - - | - - | - - | - - | + + | + + | + + | + + | + + | 0,63 |

| | | | | | | | | | | | |
|---|---|---|---|---|---|---|---|---|---|---|---|
| + = Wachstum, - = kein Wachstum | | | | | | | | | | | |

**Tabelle 8: Versuchsergebnisse der Ermittlung der MHK der Probe "Fluidextrakt V80 plus P80 (1 : 1-Gemisch)"**

| Endkonzentration in % | 10 | 5 | 2,5 | 1,25 | 0,63 | 0,31 | 0,16 | 0,08 | 0,04 | 0,02 | MHK in % |
|---|---|---|---|---|---|---|---|---|---|---|---|
| Testkeim | | | | | | | | | | | |
| *Actinomyces viscosus* | - - | - - | - - | - - | - - | + - | + + | + + | + + | + + | 0,63 |
| *Fusobacterium nucleatum* | - - | - - | - - | - - | - - | - - | + - | + + | + + | + + | 0,31 |
| *Porphyromonas gingivalis* | - - | - - | - - | - - | - - | - - | - - | + + | + + | + + | 0,16 |
| *Streptococcus mutans* | - - | - - | - - | - - | - - | - - | + - | + + | + + | + + | 0,31 |
| *Streptococcus salivarius* | - - | - - | - - | - - | - - | - - | + + | + + | + + | + + | 0,31 |
| *Streptococcus sanguinis* | - - | - - - | - - | - - | - - | - - | - + | + + | + + | + + | 0,31 |
| *Streptococcus sobrinus* | - - | - - | - - | - - | - - | + - | + + | + + | + + | + + | 0,63 |

| | | | | | | | | | | | |
|---|---|---|---|---|---|---|---|---|---|---|---|
| + = Wachstum, - = kein Wachstum | | | | | | | | | | | |

## Patentansprüche

1. Pharmazeutische Zusammensetzung zur prophylaktischen und/oder kurativen topischen Behandlung von Zahn- und Zahnfleischerkrankungen, enthaltend, neben mindestens einem pharmazeutischen Träger, eine Kombination von Eisenkraut und Spitzwegerich oder deren Inhaltsstoffen oder Extrakten in jeweils pharmazeutisch wirksamen Mengen.

2. Pharmazeutische Zusammensetzung nach Anspruch 1, enthaltend Eisenkraut und Spitzwegerich oder deren Inhaltsstoffe oder Extrakte als alleinige Wirkstoffe.

3. Pharmazeutische Zusammensetzung nach Anspruch 1 oder 2, enthaltend Eisenkraut und Spitzwegerich in Form zerkleinerter, insbesondere pulverisierter Pflanzenbestandteile und/oder in Form deren extrahierter Inhaltsstoffe und/oder in Form von Extrakten.

4. Pharmazeutische Zusammensetzung nach mindestens einem der vorangehenden Ansprüche, enthaltend Eisenkraut und Spitzwegerich in Form ihrer jeweiligen Extrakte, insbesondere in Form von Flüssig- oder Trockenextrakten, insbesondere wobei das bevorzugte Droge/Extrakt-Verhältnis 50 : 1 bis 1 : 1, besonders bevorzugt 10 : 1 bis 1 : 0,2, beträgt.

5. Pharmazeutische Zusammensetzung nach mindestens einem der vorangehenden Ansprüche, enthaltend Eisenkraut und Spitzwegerich in Form flüssiger Extrakte, insbesondere in Form alkoholischer oder alkoholisch-wäßriger Extrakte, vorzugsweise in Form ethanolischer oder ethanolisch-wäßriger Extrakte.

6. Pharmazeutische Zusammensetzung nach mindestens einem der Ansprüche 1 bis 4, enthaltend Eisenkraut und Spitzwegerich in Form fester Extrakte.

7. Pharmazeutische Zusammensetzung nach mindestens einem der vorangehenden Ansprüche, **dadurch gekennzeichnet, daß** die pharmazeutische Zusammensetzung Eisenkraut und Spitzwegerich oder deren Inhaltsstoffe oder Extrakte in Mengen von mindestens 0,1 Gew.-%, insbesondere mindestens 1 Gew.-%, vorzugsweise mindestens 5 Gew.-%, besonders bevorzugt mindestens 10 Gew.-%, jeweils berechnet auf die Masse des pflanzlichen Ausgangsmaterials und bezogen auf die pharmazeutische Zusammensetzung, enthält und/oder daß die pharmazeutische Zusammensetzung Eisenkraut und Spitzwegerich oder deren Inhaltsstoffe oder Extrakte in Mengen von bis zu 50 Gew.-%, insbesondere von bis zu 75 Gew.-%, vorzugsweise von bis zu 90 Gew.-%, besonders bevorzugt von bis zu 95 Gew.-% oder sogar mehr, jeweils berechnet auf die Masse des pflanzlichen Ausgangsmaterials und bezogen auf die pharmazeutische Zusammensetzung, enthält.

8. Pharmazeutische Zusammensetzung nach mindestens einem der vorangehenden Ansprüche, **dadurch gekennzeichnet, daß** die pharmazeutische Zusammensetzung flüssig, gelartig, pastös, salbenförmig oder fest ist.

9. Pharmazeutische Zusammensetzung nach mindestens einem der vorangehenden Ansprüche, **dadurch gekennzeichnet, daß** sie in Form von (Lutsch-/Kau-) Tabletten oder (Lutsch-/Kau-)Pastillen oder in Form einer zur topischen Applikation im Mund-/Rachenraum verwendbaren Lösung oder Dispersion, insbesondere Spül- und/oder Gurgellösung, oder in Form von zur topischen Applikation im Mund-/Rachenraum verwendbaren Salben, Cremes oder Pasten, insbesondere Zahnpasten oder Zahncremes, vorliegt.

10. Pharmazeutische Zusammensetzung nach mindestens einem der vorangehenden Ansprüche zur prophylaktischen und/oder kurativen topischen Behandlung von Zahnkaries, Gingivitis, Parodontitis und Plaque (Zahnbelag) sowie damit im Zusammenhang stehenden Symptomen bzw. Erscheinungen, wie Mundgeruch.

11. Pharmazeutische Zusammensetzung nach mindestens einem der vorangehenden Ansprüche, **dadurch gekennzeichnet, daß** sie außerdem übliche pharmazeutische Zusatz- und/oder Hilfsstoffe enthält, insbesondere Füll-, Streck-, Binde-, Netz-, Stabilisierungs-, Färbe-, Puffer-, Riech-, Geschmacks- und/oder Konservierungsstoffe.

12. Verwendung einer Kombination von Eisenkraut und Spitzwegerich oder deren jeweiligen Inhaltsstoffen oder Extrakte zur Herstellung eines pharmazeutischen Mittels zur prophylaktischen und/oder kurativen topischen Behandlung von Zahn- und Zahnfleischerkrankungen, insbesondere Zahnkaries, Gingivitis, Parodontitis und Plaque (Zahnbelag) sowie damit im Zusammenhang stehenden Symptomen bzw. Erscheinungen, wie Mundgeruch.

13. Verwendung nach mindestens einem der vorangehenden Ansprüche, **dadurch gekennzeichnet, daß** Eisenkraut und Spitzwegerich oder deren jeweilige Inhaltsstoffe oder Extrakte in pharmazeutisch wirksamen Mengen angewandt oder eingesetzt werden.

14. Verwendung nach mindestens einem der vorangehenden Ansprüche, **dadurch gekennzeichnet, daß** Eisenkraut und Spitzwegerich oder deren jeweilige Inhaltsstoffe oder Extrakte in Form einer pharmazeutischen Zusammensetzung oder medizinischen Zubereitung eingesetzt werden, insbesondere wobei die pharmazeutische Zusammensetzung oder medizinische Zubereitung außerdem mindestens einen pharmazeutischen Träger enthält und/oder insbesondere wobei die pharmazeutische Zusammensetzung oder medizinische Zubereitung flüssig, gelartig, pastös, salbenformig oder fest ist und/oder insbesondere wobei die pharmazeutische Zusammensetzung oder medizinische Zubereitung Eisenkraut und Spitzwegerich oder deren Inhaltsstoffe oder Extrakte, jeweils berechnet auf die Masse des pflanzlichen Ausgangsmaterials und bezogen auf die pharmazeutische Zusammensetzung, in Mengen von mindestens 0,1 Gew.-%, insbesondere mindestens 1 Gew.-%, vorzugsweise mindestens 5 Gew.-%, besonders bevorzugt mindestens 10 Gew.-%, und/oder in Mengen von bis zu 50 Gew.-%, insbesondere von bis zu 75 Gew.-%, vorzugsweise von bis zu 90 Gew.-%, besonders bevorzugt von bis zu 95 Gew.-% oder sogar mehr, enthält.

15. Verwendung nach mindestens einem der vorangehenden Ansprüche, **dadurch gekennzeichnet, daß** Eisenkraut und Spitzwegerich in Form zerkleinerter, insbesondere pulverisierter Pflanzenbestandteile bzw. Pflanzenmaterialien und/oder in Form deren extrahierter Inhaltsstoffe und/oder in Form von Extrakten eingesetzt werden.

16. Verwendung nach mindestens einem der vorangehenden Ansprüche, **dadurch gekennzeichnet, daß** Eisenkraut und Spitzwegerich oder deren Inhaltsstoffe oder Extrakte als alleinige Wirkstoffe eingesetzt werden.

17. Verwendung nach mindestens einem der vorangehenden Ansprüche, **dadurch gekennzeichnet, daß** Eisenkraut und Spitzwegerich in Form ihrer jeweiligen Extrakte, insbesondere in Form von Flüssig- oder Trockenextrakten, eingesetzt werden, insbesondere wobei das bevorzugte Droge/Extrakt-Verhältnis 50 : 1 bis 1 : 1, besonders bevorzugt 10 : 1 bis 1 : 0,2, beträgt.

18. Verwendung nach mindestens einem der vorangehenden Ansprüche, **dadurch gekennzeichnet, daß** Eisenkraut und Spitzwegerich in Form flüssiger Extrakte, insbesondere in Form alkoholischer oder alkoholisch-wäßriger Extrakte, vorzugsweise in Form ethanolischer oder ethanolisch-wäßriger Extrakte, eingesetzt werden.

19. Verwendung nach mindestens einem der Ansprüche 12 bis 17, **dadurch gekennzeichnet, daß** Eisenkraut und Spitzwegerich in Form fester Extrakte eingesetzt werden.

20. Verwendung nach mindestens einem der vorangehenden Ansprüche, **dadurch gekennzeichnet, daß** Eisenkraut und Spitzwegerich oder deren jeweilige Inhaltsstoffe oder Extrakte in Form von (Lutsch-/Kau-)Tabletten oder (Lutsch-/Kau-) Pastillen oder in Form einer zur topischen Applikation im Mund-/Rachenraum verwendbaren Lösung oder Dispersion, insbesondere Spül- und/oder Gurgellösung, oder in Form von zur topischen Applikation im Mund-/Rachenrauzn verwendbaren Salben, Cremes, Gelen oder Pasten, insbesondere Zahnpasten oder Zahncremes, eingesetzt werden.

21. Verwendung nach mindestens einem der vorangehenden Ansprüche, **dadurch gekennzeichnet, daß** Eisenkraut und Spitzwegerich oder deren jeweilige Inhaltsstoffe oder Extrakte zusammen mit üblichen pharmazeutischen Zusatz- und/oder Hilfsstoffen, insbesondere Füll-, Streck-, Binde-, Netz-, Stabilisierungs-, Färbe-, Puffer-, Riech-,Geschmacks- und/oder Konservierungsstoffen, eingesetzt werden.

## Claims

1. Pharmaceutical composition for the prophylactic and/or curative topical treatment of dental and periodontal diseases comprising, in addition to at least one pharmaceutical excipient, a combination of vervain and ribwort or their constituents or extracts, in each case in pharmaceutically active amounts.

2. Pharmaceutical composition according to Claim 1, containing vervain and ribwort or their constituents or extracts as the sole active ingredients.

3. Pharmaceutical composition according to Claim 1 or 2, containing vervain and ribwort in the form of comminuted, in particular powdered, plant components and/or in the form of their extracted constituents and/or in the form of extracts.

4. Pharmaceutical composition according to at least one of the preceding claims, containing vervain and ribwort in the form of their respective extracts, in particular in the form of liquid or dry extracts, the preferred herb/extract ratio being in particular 50:1 to 1:1, especially preferably 10:1 to 1:0.2.

5. Pharmaceutical composition according to at least one of the preceding claims, containing vervain and ribwort in the form of liquid extracts, in particular in the form of alcoholic or alcoholic-aqueous extracts, preferably in the form of ethanolic or ethanolic-aqueous extracts.

6. Pharmaceutical composition according to at least one of Claims 1 to 4, containing vervain and ribwort in the form of solid extracts.

7. Pharmaceutical composition according to at least one of the preceding claims, **characterized in that** the pharmaceutical composition contains vervain and ribwort or their constituents or extracts in amounts of at least 0.1 % by weight, in particular at least 1% by weight, preferably at least 5% by weight, especially preferably at least 10% by weight, in each case based on the amount of the original plant material and based on the pharmaceutical composition, and/or that the pharmaceutical composition contains vervain and ribwort or their constituents or extracts in amounts of from up to 50% by weight, in particular of up to 75% by weight, preferably of up to 90% by weight, especially preferably of up to 95% by weight or even more, in each case based on the amount of original plant material and based on the pharmaceutical composition.

8. Pharmaceutical composition according to at least one of the preceding claims, **characterized in that** the pharmaceutical composition is liquid, gel-like, pasty, ointment-like or solid.

9. Pharmaceutical composition according to at least one of the preceding claims, **characterized in that** it exists in the form of lozenges/chewable tablets or (chewable) pastilles or in the form of a solution or dispersion which can be used for topical application in the oropharyngeal cavity, in particular a wash and/or gargle solution, or in the form of ointments, creams or pastes which can be used for topical application in the oropharyngeal cavity, in particular toothpastes.

10. Pharmaceutical composition according to at least one of the preceding claims for the prophylactic and/or curative topical treatment of dental decay, gingivitis, periodontosis and plaque (dental deposit) and associated symptoms or phenomena such as halitosis.

11. Pharmaceutical composition according to at least one of the preceding claims, **characterized in that** it additionally contains conventional pharmaceutical additives and/or auxiliaries, in particular fillers, extenders, binders, wetting agents, stabilizers, colorants, buffers, aroma chemicals, flavourings and/or preservatives.

12. Use of a combination of vervain and ribwort or their respective constituents or extracts for the preparation of a pharmaceutical product for the prophylactic and/or curative topical treatment of dental and periodontal diseases, in particular dental decay, gingivitis, periodontosis and plaque (dental deposit) and associated symptoms or phenomena such as halitosis.

13. Use according to at least one of the preceding claims, **characterized in that** vervain and ribwort or their respective constituents or extracts are employed or utilized in pharmaceutically effective amounts.

14. Use according to at least one of the preceding claims, **characterized in that** vervain and ribwort or their respective constituents or extracts are employed in the form of a pharmaceutical composition or medicinal preparation, in particular where the pharmaceutical composition or medicinal preparation additionally contains at least one pharmaceutical excipient and/or in particular where the pharmaceutical composition or medicinal preparation is liquid, gel-like, pasty, ointment-like or solid and in particular where the pharmaceutical composition or medicinal preparation contains vervain and ribwort or their constituents or extracts in amounts of at least 0.1% by weight, in particular at least 1% by weight, preferably at least 5% by weight, particularly preferably at least 10% by weight, and/or in amounts of up to 50% by weight, in particular of up to 75% by weight, preferably of up to 90% by weight, especially preferably of up to 95% by weight or even more, in each case based on the amount of the original plant material and based on the pharmaceutical composition.

15. Use according to at least one of the preceding claims, **characterized in that** vervain and ribwort are employed in the form of comminuted, in particular powdered, plant components or plant materials and/or in the form of their extracted constituents and/or in the form of extracts.

16. Use according to at least one of the preceding claims, **characterized in that** vervain and ribwort or their constituents or extracts are employed as the sole active ingredients.

17. Use according to at least one of the preceding claims, **characterized in that** vervain and ribwort in the form of their respective extracts, in particular in the form of liquid or dry extracts, the preferred herb/extract ratio being in particular 50:1 to 1:1, especially preferably 10:1 to 1:0.2.

18. Use according to at least one of the preceding claims, **characterized in that** vervain and ribwort are employed in the form of liquid extracts, in particular in the form of alcoholic or alcoholic-aqueous extracts, preferably in the form of ethanolic or ethanolic-aqueous extracts.

19. Use according to at least one of Claims 12 to 17, **characterized in that** vervain and ribwort are employed in the form of solid extracts.

20. Use according to at least one of the preceding claims, **characterized in that** vervain and ribwort or their respective constituents or extracts are employed in the form of lozenges/ chewable tablets or (chewable) pastilles or in the form of a solution or dispersion which can be used for topical application in the oropharyngeal cavity, in particular a wash and/or gargle solution, or in the form of ointments, creams or pastes which can be used for topical application in the oropharyngeal cavity, in particular toothpastes.

21. Use according to at least one of the preceding claims, **characterized in that** vervain and ribwort or their respective constituents or extracts are employed together with customary pharmaceutical additives and/or auxiliaries, in particular fillers, extenders, binders, wetting agents, stabilizers, colorants, buffers, aroma chemicals, flavourings and/or preservatives.

## Revendications

1. Composition pharmaceutique pour le traitement prophylactique et/ou curatif d'affections dentaires et des gencives, contenant, outre au moins un véhicule pharmaceutique, une association de verveine officinale et de plantain lancéolé ou de leurs composants ou extraits en quantités respectives pharmaceutiquement efficaces.

2. Composition pharmaceutique selon la revendication 1, contenant en tant que seuls principes actifs de la verveine officinale et du plantain lancéolé ou leurs composants ou extraits.

3. Composition pharmaceutique selon la revendication 1 ou 2, contenant de la verveine officinale ou du plantain lancéolé, sous forme de constituants végétaux fragmentés, en particulier pulvérisés et/ou sous forme de leurs composants extraits et/ou sous forme d'extraits.

4. Composition pharmaceutique selon au moins l'une des revendications précédentes, contenant de la verveine officinale et du plantain lancéolé sous forme de leurs extraits respectifs, en particulier sous forme d'extraits secs ou liquides, en particulier le rapport médicament/extrait préféré allant de 50:1 à 1:1, de façon particulièrement préférée de 10:1 à 1:0,2.

5. Composition pharmaceutique selon au moins l'une des revendications précédentes, contenant de la verveine officinale et du plantain lancéolé sous forme d'extraits liquides, en particulier sous forme d'extraits alcooliques ou aqueux-alcooliques, de préférence sous forme d'extraits éthanoliques ou aqueux-éthanoliques.

6. Composition pharmaceutique selon au moins l'une des revendications 1 à 4, contenant de la verveine officinale et du plantain lancéolé sous forme d'extraits solides.

7. Composition pharmaceutique selon au moins l'une des revendications précédentes, **caractérisée en ce que** la composition pharmaceutique contient de la verveine officinale et du plantain lancéolé ou leurs composants ou extraits en quantités d'au moins 0,1 % en poids, en particulier d'au moins 1 % en poids, de préférence d'au moins 5 % en poids, de façon particulièrement préférée d'au moins 10 % en poids, chaque fois calculé sur la masse du produit végétal de départ et par rapport à la composition pharmaceutique et/ou en ce que la composition pharmaceutique contient de la verveine officinale et du plantain lancéolé ou leurs composants ou extraits en quantités allant jusqu'à 50 % en poids, en particulier jusqu'à 75 % en poids, de préférence jusqu'à 90 % en poids, de façon particulièrement préférée jusqu'à 95 % en poids ou même plus, chaque fois calculé sur la masse du produit végétal de départ et par rapport à la composition pharmaceutique.

8. Composition pharmaceutique selon au moins l'une des revendications précédentes, **caractérisée en ce que** la composition pharmaceutique est liquide, gélatineuse, pâteuse, sous forme de pommade ou solide.

9. Composition pharmaceutique selon au moins l'une des revendications précédentes, **caractérisée en ce qu'**elle se trouve sous forme de comprimés (à sucer/à mâcher) ou de pastilles (à sucer/à mâcher)ou sous forme d'une solution ou dispersion utilisable pour l'application locale dans la cavité buccale/pharyngée, en particulier d'une solution de rinçage ou pour gargarisme, ou sous forme de pommades, crèmes ou pâtes utilisables pour l'application locale dans la cavité buccale/pharyngée, en particulier de pâtes dentifrices ou de crèmes dentaires.

10. Composition pharmaceutique selon au moins l'une des revendications précédentes, pour le traitement prophylactique et/ou curatif de caries, de gingivite, de parodontose et de la plaque (dépôt sur les dents) ainsi que des symptômes ou phénomènes en relation avec celles-ci, tels que l'haleine chargée.

11. Composition pharmaceutique selon au moins l'une des revendications précédentes, **caractérisée en ce qu'**elle contient en outre des additifs et/ou adjuvants pharmaceutiques usuels, en particulier des excipients, diluants, liants, agents mouillants, stabilisants, colorants, tampons, parfums, arômes et/ou conservateurs.

12. Utilisation d'une association de verveine officinale et de plantain lancéolé ou de leurs composants ou extraits respectifs, pour la fabrication d'un produit pharmaceutique destiné au traitement prophylactique et/ou curatif d'affections dentaires et des gencives, en particulier de caries, de gingivite, de parodontose et de la plaque (dépôt sur les dents) ainsi que des symptômes ou phénomènes en relation avec celles-ci, tels que l'haleine chargée.

13. Utilisation selon au moins l'une des revendications précédentes, **caractérisée en ce que** la verveine officinale et le plantain lancéolé ou leurs composants ou extraits respectifs sont utilisés ou appliqués en quantités pharmaceutiquement efficaces.

14. Utilisation selon au moins l'une des revendications précédentes, **caractérisée en ce que** la verveine officinale et le plantain lancéolé ou leurs composants ou extraits respectifs sont utilisés sous forme d'une composition pharmaceutique ou d'une préparation médicale, en particulier la composition pharmaceutique ou la préparation médicale contenant en outre au moins un véhicule pharmaceutique et/ou en particulier la composition pharmaceutique ou la préparation médicale étant liquide, gélatineuse, pâteuse, sous forme de pommade ou solide et/ou en particulier la composition pharmaceutique ou la préparation médicale contenant de la verveine officinale et du plantain lancéolé ou leurs composants ou extraits respectivement en quantités d'au moins 0,1 % en poids, en particulier d'au moins 1 % en poids, de préférence d'au moins 5 % en poids, de façon particulièrement préférée d'au moins 10 % en poids, et/ou en quantités allant jusqu'à 50 % en poids, en particulier jusqu'à 75 % en poids, de préférence jusqu'à 90 % en poids, de façon particulièrement préférée jusqu'à 95 % en poids ou même plus, chaque fois calculé sur la masse du produit végétal de départ et par rapport à la composition pharmaceutique.

15. Utilisation selon au moins l'une des revendications précédentes, **caractérisée en ce que** la verveine officinale et le plantain lancéolé sont utilisés sous forme de constituants végétaux fragmentés, en particulier pulvérisés, ou de matières végétales fragmentées, en particulier pulvérisées et/ou sous forme de leurs composants extraits et/ou sous forme d'extraits.

16. Utilisation selon au moins l'une des revendications précédentes, **caractérisée en ce que** la verveine officinale et le plantain lancéolé ou leurs composants ou extraits sont utilisés en tant que seuls principes actifs.

17. Utilisation selon au moins l'une des revendications précédentes, **caractérisée en ce que** la verveine officinale et le plantain lancéolé sont utilisés sous forme de leurs extraits respectifs, en particulier sous forme d'extraits secs ou liquides, en particulier le rapport médicament/extrait préféré allant de 50:1 à 1:1, de façon particulièrement préférée de 10:1 à 1:0,2.

18. Utilisation selon au moins l'une des revendications précédentes, **caractérisée en ce que** la verveine officinale et le plantain lancéolé sont utilisés sous forme d'extraits liquides, en particulier sous forme d'extraits alcooliques ou aqueux-alcooliques, de préférence sous forme d'extraits éthanoliques ou aqueux-éthanoliques.

19. Utilisation selon au moins l'une des revendications 12 à 17, **caractérisée en ce que** la verveine officinale et le plantain lancéolé sont utilisés sous forme d'extraits solides.

20. Utilisation selon au moins l'une des revendications précédentes, **caractérisée en ce que** la verveine officinale et le plantain lancéolé ou leurs composants ou extraits respectifs sont utilisés sous forme de comprimés (à sucer/à mâcher) ou de pastilles (à sucer/à mâcher) ou sous forme d'une solution ou dispersion utilisable pour l'application locale dans la cavité buccale/pharyngée, en particulier de solution de rinçage et/ou pour gargarisme, ou sous forme de pommades, crèmes ou pâtes utilisables pour l'application locale dans la cavité buccale/pharyngée, en particulier de pâtes dentifrices ou de crèmes dentaires.

21. Utilisation selon au moins l'une des revendications précédentes, **caractérisée en ce que** la verveine officinale et le plantain lancéolé ou leurs composants ou extraits respectifs sont utilisés conjointement avec des additifs et/ou adjuvants pharmaceutiques usuels, en particulier des excipients, diluants, liants, agents mouillants, stabilisants, colorants, tampons, parfums, arômes et/ou conservateurs.
